(19) **Europäisches Patentamt / European Patent Office / Office européen des brevets**

(11) **EP 4 628 902 A1**

(12) **EUROPEAN PATENT APPLICATION**
published in accordance with Art. 153(4) EPC

(43) Date of publication:
08.10.2025 Bulletin 2025/41

(21) Application number: 23897394.5

(22) Date of filing: 07.11.2023

(51) International Patent Classification (IPC):
$G01N\ 35/08^{(2006.01)}$    $B01J\ 19/00^{(2006.01)}$
$B32B\ 5/18^{(2006.01)}$    $G01N\ 37/00^{(2006.01)}$

(52) Cooperative Patent Classification (CPC):
B01J 19/00; B32B 5/18; G01N 35/08; G01N 37/00

(86) International application number:
PCT/JP2023/039971

(87) International publication number:
WO 2024/116740 (06.06.2024 Gazette 2024/23)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 28.11.2022 JP 2022188860

(71) Applicant: Dexerials Corporation
Shimotsuke-shi, Tochigi 323-0194 (JP)

(72) Inventor: MONJU, Takuya
Shimotsuke-shi, Tochigi 323-0194 (JP)

(74) Representative: Ter Meer Steinmeister & Partner
Patentanwälte mbB
Nymphenburger Straße 4
80335 München (DE)

(54) **SHEET-LIKE STRUCTURE AND METHOD FOR USING SAME**

(57)    A sheet-like structure includes a porous structure layer and a support layer. In the porous structure layer, at least a part of a flow path having a porous structure capable of passing a fluid through is exposed at a surface of the porous structure layer. The flow path has a separation portion capable of separating adjacent portions of the flow path to block flow of the fluid in the flow path.

FIG.1A

**Description**

TECHNICAL FIELD

The present invention relates to a sheet-like structure and a method of using the sheet-like structure.

BACKGROUND ART

Inspection devices that enable simple and rapid diagnosis in daily life or clinical practice have been developed. A representative example of the inspection device is a pregnancy test kit. When a test liquid including a target substance, such as an antigen, is introduced into the inspection device, the test liquid flows through a flow path within the inspection device. Then, a labeling medium such as an antibody, which has been disposed in the flow path in advance, reacts with the target substance included in the testing liquid to develop a color (to generate a color) so that the presence of the target substance can be confirmed.

Inspection chips, which are one example of the inspection devices, are also referred to as "u-PADs (microfluidic paper-based analytical devices)." The inspection chips have many advantages, such as (1) low cost, (2) pumpless, (3) no need of complex devices, and (4) easy disposal, and therefore studies for improving the inspection chips have been conducted worldwide.

Various inspection chips (inspection devices) have been already reported. For example, an inspection device in which liquid absorption pads (paper discs) are respectively disposed between sheets of multiple paper chips is proposed for the purpose of ensuring a sufficient reaction time between a phosphorus-based agricultural chemical serving as a test sample and acetylcholine esterase (AChE) that reacts with the phosphorus-based agricultural chemical, and providing a device having a high coloring intensity of a coloring reaction and achieving high accuracy (see Non-Patent Document 1). The above inspection device ensures a reaction time between the test sample and AChE by utilizing a delay in flow of a fluid caused by the liquid absorption pads. Moreover, an inspection chip in which a three-dimensional flow path is formed within one sheet-like material is proposed for the purpose of effectively minimizing coloring unevenness (see Patent Document 1).

CITATION LIST

PATENT DOCUMENTS

Patent Document 1: Japanese Unexamined Patent Application Publication No. 2021-175970

NON-PATENT DOCUMENTS

NON-PATENT DOCUMENT 1: Quoc Trung Hua et al., analytical sciences, April, 2019, Vol. 35, pp. 393-399

SUMMARY OF THE INVENTION

TECHNICAL PROBLEM

However, the inspection device involving the technology described in Non-Patent Document 1 above allows flow of a fluid that has not sufficiently proceeded with a primary reaction (reaction between a test sample and AChE), and multiple substrates in which the flow path is formed are bonded together with a double-sided tape. Therefore, it is necessary to fill the gap between the substrates corresponding to the thickness of the double-sided tape, and there is a concern that an assembling process may become complicated. Moreover, regarding the inspection chip described in Patent Document 1 above, a test sample and an inspection reagent (e.g., antigen and antibody) needs to be mixed in advance and reacted before adding the test sample and the inspection reagent to the inspection chip because a flow rate of a fluid in the inspection chip is fast and a reaction time for a primary reaction cannot be sufficiently ensured. There is therefore a need for improving disposal of a container used for mixing and reaction, contamination of a test sample, simplicity in procedure, and the like.

The present invention aims to solve the above various problems existing in the related art, and to achieve the following object. Specifically, an object of the present invention is to provide a sheet-like structure that can ensure a sufficient reaction time, and can improve accuracy of quantitative analyses.

SOLUTION TO THE PROBLEM

Means for solving the above problems are as follows.

<1> A sheet-like structure including:

a porous structure layer in which at least a part of a flow path having a porous structure capable of passing a fluid through is exposed at a surface of the porous structure layer; and
a support layer,

wherein the flow path has a separation portion capable of separating adjacent portions of the flow path to block flow of the fluid in the flow path.
<2> The sheet-like structure according to <1>,
wherein the separation portion is a cut portion that cuts off the flow path in a direction intersecting with a flow direction of the fluid in the flow path.
<3> The sheet-like structure according to <1> or <2>,
wherein at least a part of the part of the flow path exposed to the surface is a fluid inlet portion.
<4> The sheet-like structure according to <3>,
wherein the separation portion is capable of separating the fluid inlet portion from a portion of the flow path adjacent to the fluid inlet portion so that a fluid that has entered from the fluid inlet portion is blocked from flowing into the portion of the flow path adjacent to the fluid inlet portion.
<5> The sheet-like structure according to <3> or <4>,
wherein the support layer is arranged on a region of the porous structure layer in which the fluid inlet portion is not located.
<6> The sheet-like structure according to any one of <1> to <5>,
wherein the support layer is impermeable to water.
<7> The sheet-like structure according to any one of <1> to <6>,
wherein an exposed shape of the flow path that is exposed at one surface of the porous structure layer is different from an exposed shape of the flow path that is exposed at the other surface of the porous structure layer.
<8> The sheet-like structure according to any one of <3> to <5>,
wherein at least part of the part of the flow path exposed at the surface is a detection portion, and the detection portion is arranged in a position of the flow path different from a position of the fluid inlet portion.
<9> A method of using the sheet-like structure of any one of <1> to <8>, the method including:

allowing a fluid to enter a first flow path position at one side with respect to the separation portion;
separating the first flow path from a second flow path at the separation portion to block flow of the fluid so that the fluid that has entered from the first flow path does not flow into the second flow path positioned at the other side with respect to the separation portion; and
after a predetermined time has elapsed, bringing the first flow path and the second flow path into contact with each other at the separation portion to allow the fluid to flow between the first flow path and the second flow path.

<10> The method of using the sheet-like structure according to <9>,
wherein the first flow path is a fluid inlet portion, and a detection portion is arranged at a part of the second flow path.

EFFECTS OF THE INVENTION

According to the present invention, a sheet-like structure that can ensure a sufficient reaction time and improve accuracy of quantitative analyses can be provided.

BRIEF DESCRIPTION OF THE DRAWINGS

[Fig. 1A] Fig. 1A is a schematic perspective view of a sheet-like structure of a first embodiment.
[Fig. 1B] Fig. 1B is a schematic cross-sectional view of the sheet-like structure of Fig. 1A cut along the line a-a'.
[Fig. 2A] Fig. 2A is an explanatory view for explaining a function of a separation portion.
[Fig. 2B] Fig. 2B is an explanatory view for explaining another function of the separation portion.
[Fig. 3A] Fig. 3A is an explanatory view for explaining an example of a distribution of a support layer.

[Fig. 3B] Fig. 3B is an explanatory view for explaining another example of the distribution of the support layer.

[Fig. 3C] Fig. 3C is an explanatory view for explaining yet another example of the distribution of the support layer.

[Fig. 4A] Fig. 4A is a schematic cross-sectional view of a sheet-like structure of a second embodiment.

[Fig. 4B] Fig. 4B is a schematic plan view of the porous structure layer of Fig. 4A as viewed in the α direction.

[Fig. 4C] Fig. 4C is a schematic plan view of the porous structure layer of Fig. 4A as viewed in the β direction.

[Fig. 5A] Fig. 5A is a schematic cross-sectional view of a sheet-like structure of a third embodiment.

[Fig. 5B] Fig. 5B is a schematic plan view of the porous structure layer of Fig. 5A as viewed in the α direction.

[Fig. 5C] Fig. 5C is a schematic plan view of the porous structure layer of Fig. 5A as viewed in the β direction.

[Fig. 6A] Fig. 6A is a schematic cross-sectional view of a sheet-like structure of a fourth embodiment.

[Fig. 6B] Fig. 6B is a schematic plan view of the porous structure layer of Fig. 6A as viewed in the α direction.

[Fig. 6C] Fig. 6C is a schematic plan view of the porous structure layer of Fig. 6A as viewed in the β direction.

[Fig. 7A] Fig. 7A is a schematic plan view of a porous structure layer of a sheet-like structure of a fifth embodiment as viewed in the α direction.

[Fig. 7B] Fig. 7B is a schematic plan view of the porous structure layer of the sheet-like structure of the fifth embodiment as viewed in the β direction.

[Fig. 8A] Fig. 8A is a schematic plan view of a porous structure layer of a sheet-like structure of a sixth embodiment as viewed in the α direction.

[Fig. 8B] Fig. 8B is a schematic plan view of the porous structure layer of the sheet-like structure of the sixth embodiment as viewed in the β direction.

[Fig. 9A] Fig. 9A is a schematic plan view of a porous structure layer of a sheet-like structure of a seventh embodiment as viewed in the α direction.

[Fig. 9B] Fig. 9B is a schematic plan view of the porous structure of the sheet-like structure of the seventh embodiment as viewed in the β direction.

[Fig. 10A] Fig. 10A is a schematic plan view of a porous structure layer of a sheet-like structure of an eighth embodiment as viewed in the α direction.

[Fig. 10B] Fig. 10B is a schematic plan view of the porous structure layer of the sheet-like structure of the eighth embodiment as viewed in the β direction.

[Fig. 11A] Fig. 11A is a schematic plan view of a porous structure layer of a sheet-like structure of a ninth embodiment as viewed in the α direction.

[Fig. 11B] Fig. 11B is a schematic plan view of the porous structure layer of the sheet-like structure of the ninth embodiment as viewed in the β direction.

[Fig. 12A] Fig. 12A is a schematic plan view of a porous structure layer of a sheet-like structure of a tenth embodiment as viewed in the α direction.

[Fig. 12B] Fig. 12B is a schematic plan view of the porous structure layer of the sheet-like structure of the tenth embodiment as viewed in the β direction.

[Fig. 13A] Fig. 13A is an explanatory view for explaining a specific flow path shape in Examples.

[Fig. 13B] Fig. 13B is an explanatory view for explaining a specific flow path shape in Examples.

[Fig. 13C] Fig. 13C is an explanatory view for explaining a missing flow path shape in Examples.

[Fig. 13D] Fig. 13D is an explanatory view for explaining a missing flow path shape in Examples.

[Fig. 14A] Fig. 14A is an explanatory view for explaining a single-side or double-side printed flow path in Examples.

[Fig. 14B] Fig. 14B is a cross-sectional photograph of the single-side printed flow path of Fig. 14A cut along the line f-f'.

[Fig. 14C] Fig. 14C is a cross-sectional photograph of the double-side printed flow path of Fig. 14A cut along the line f-f'.

[Fig. 15] Fig. 15 is a schematic cross-sectional view for explaining the porous structure layer in Examples.

[Fig. 16] Fig. 16 is a schematic cross-sectional view for explaining the sheet-like structure in Examples.

[Fig. 17] Fig. 17 is a schematic cross-sectional view for explaining the sheet-like structure in Examples.

[Fig. 18A] Fig. 18A is a graph depicting the measurement results of the quantitative analysis in Examples.

[Fig. 18B] Fig. 18B is a graph depicting the measurement results of the quantitative analysis in Examples.

## DESCRIPTION OF EMBODIMENTS

(Laminate structure)

The sheet-like structure of the present invention includes a porous structure layer and a support layer. In the porous structure layer, at least a part of a flow path that is a porous structure capable of passing a fluid through is exposed to a surface of the porous structure layer. The flow path has a separation portion capable of separating adjacent portions of the flow path to block flow of the fluid in the flow path.

The present invention will be described in detail based on several embodiments hereinafter, but the present invention is not limited to the following descriptions of the embodiments in any way.

<First embodiment>

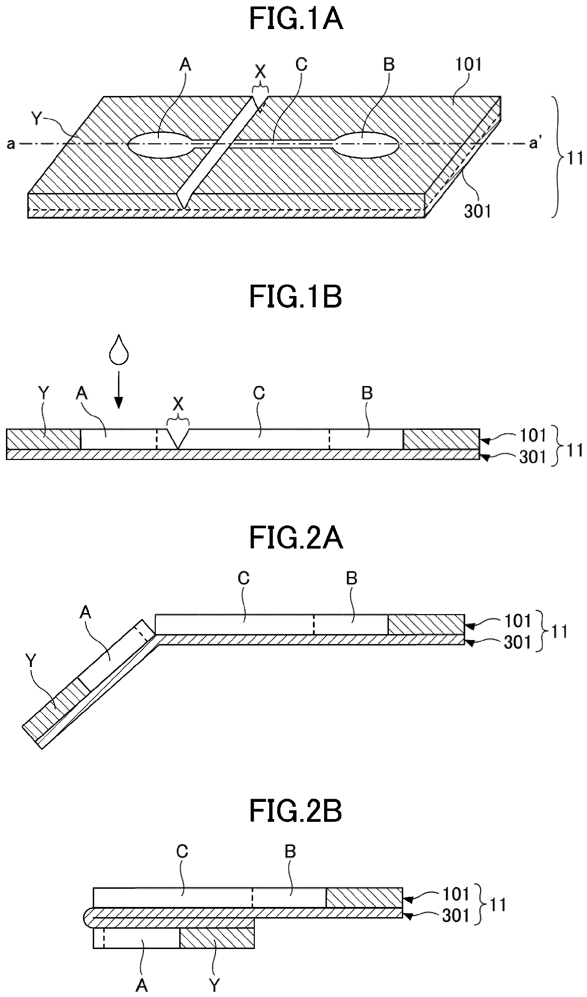

Fig. 1A is a schematic perspective view of the sheet-like structure of the first embodiment. Fig. 1B is a schematic cross-sectional view of the sheet-like structure of Fig. 1A cut along the line a-a'.

The sheet-like structure 11 includes a porous structure layer 101, a support layer 301, and a separation portion **X.** Although the details will be described later, the sheet-like structure 11 has the above configuration, and therefore flow of a fluid in the flow path of the porous structure layer 101 can be blocked, so that a reaction time of, for example, a test sample and a reaction reagent can be sufficiently ensured.

-Porous structure layer-

In the porous structure layer 101 of the sheet-like structure 11, a flow path A, a flow path B, a flow path C, which are each formed of a porous structure through which a fluid can flow due to a capillary action or the like, and a non-flow path Y that is a region other than the flow paths A to C are provided. The flow path C is connected to both the flow path A and the flow path B, and a fluid can flow in the order of the flow path A, the flow path C, and the flow path B, or in the order of the flow path B, the flow path C, and the flow path A. In the case where a fluid is allowed to enter the flow path A as illustrated in Fig. 1B, for example, the fluid flows in the order of the flow path A, the flow path C, and the flow path B.

The flow path of the porous structure is not limited as long as the flow path is formed in a manner such that at least a part of the flow path is exposed at a surface of the porous structure layer. The flow path may be formed in a manner such that the flow path is exposed at the entire surface of the porous structure layer. Moreover, the porous structure (flow path) may be formed at only a part of the porous structure layer, or in an entirety of the porous structure layer.

In the porous structure layer, at least a part of the flow path exposed at the surface is preferably a fluid inlet portion. For example, the flow path A from which a fluid is allowed to enter can be set as a fluid inlet portion in the porous structure layer illustrated in Fig. 1B. Moreover, in the porous structure layer, at least a part of the flow path exposed at the surface is preferably a detection portion, and the detection portion is preferably arranged at a position of the flow path, which is different from the fluid inlet portion. For example, the flow path B can be set as the detection portion in the porous structure layer illustrated in Fig. 1B.

The "fluid" is not particularly limited as long as the fluid is a fluid that can be passed through the flow path composed of the porous structure by a capillary action or the like, and may be appropriately selected according to the intended purpose. In the case where the sheet-like structure of the present invention is applied for an inspection device, examples of the fluid include solutions including a test sample and a reaction reagent that reacts with the test sample, and the like.

A viscosity of the fluid is not particularly limited as long as the viscosity of the fluid is a viscosity at which the fluid can be passed through the flow path composed of the porous structure, and may be appropriately adjusted according to the intended purpose.

The "porous structure" is a structure including multiple communicating pores, and may be generally referred to as a co-continuous structure or a monolithic structure. In the porous structure, continuously connected pores are three-dimensionally spread, thereby allowing a fluid to penetrate (i.e., a capillary action).

A cross-sectional shape of each pore in the porous structure can be appropriately set considering physical properties of a fluid, such as viscosity or the like. Examples of the cross-sectional shape of each pore include substantially circular shapes, substantially elliptical shapes, substantially polygonal shape, and the like. A size of the pores in the porous structure is not particularly limited, and may be appropriately selected according to the intended purpose. The cross-sectional shapes and size of the pores can be determined, for example, from a cross-sectional photograph captured by a scanning electron microscope (SEM) or the like.

The porosity of the porous structure can be appropriately set considering physical properties of a fluid, such as viscosity, or the like. A method of measuring the porosity is not particularly limited. Examples of the method include a method in which a porous structure is filled with unsaturated fatty acid (commercially available butter), osmium staining is performed, followed by cutting out an inner cross-sectional structure by a focused ion beam (FIB), and a porosity is measured using a scanning electron microscope (SEM), and the like.

The distribution of the pores within the porous structure can be appropriately set considering physical properties of a fluid, such as viscosity or the like, as long as a fluid can flow through the porous structure. The pores are preferably uniformly distributed within the flow path region.

A shape of each of the flow paths A to C in a plan view is not particularly limited as long as a fluid can be passed through the flow path, and may be appropriately selected according to the intended purpose. Examples of the shape include circular shapes, elliptical shapes, square shapes, rectangular shapes, and the like.

A diameter of each of the flow paths A and B is not particularly limited, and may be appropriately selected according to the intended purpose. For example, the diameter can be set to 3 mm or greater and 10 mm or less. A flow path width of the flow path C is not particularly limited, and may be appropriately selected according to the intended purpose. For example, the flow path width can be set to 1 mm or greater and 5 mm or less.

The material M of each of the flow paths A to C is not particularly limited as long as the material M has a porous structure through which a fluid can flow, and may be appropriately selected according to the intended purpose. Examples of the material M include paper, such as filter paper, non-woven fabrics, nitrocellulose, polypropylene, and the like. Among the above materials, filter paper is more preferred in view of simplicity and low cost.

The non-flow path Y is a region of the porous structure layer excluding the flow paths A to C, specifically, a region where flow of a fluid does not occur.

The material M' of the non-flow path Y is not particularly limited as long as flow of a fluid does not occur, and may be appropriately selected according to the intended purpose. For example, the material M' can be obtained by impregnating the material M with a hydrophobic material. The hydrophobic material is preferably a material having a melting point of 90°C or lower in view of easy production of the porous structure layer. Examples of the material **M'** include wax, compositions including wax, and the like. A viscosity adjusting component **(e.g., a** resin), dispersion aids, fillers, and the like can be appropriately added to the hydrophobic material.

In the case where the material M is impregnated with the hydrophobic material, the hydrophobic material is preferably melted by heating in advance. The heating temperature can be appropriately set considering a melting point of the hydrophobic material or a melting point of a viscosity adjusting component.

A viscosity of the hydrophobic material when melted can be appropriately set so that the porous structure layer can be impregnated with the hydrophobic material as desired, considering an average thickness, basis weight (density), or the like of the porous structure layer.

In the case where the material M of the flow path is impregnated with the hydrophobic material, an impregnation ratio of the hydrophobic material relative to the material M is preferably within the range of 14% or greater and 32% or less.

By producing the porous structure layer to have the impregnation ratio of 14% or greater, a wall surface (an interface between the material M and the material M') of the flow path becomes sufficiently smooth, so that flow of a fluid, for example, from the flow path to another flow path, can be made smoother. By producing the porous structure layer to have the impregnation ratio of 32% or less, a problem, such as blockage, is sufficiently avoided when the material M is impregnated with the hydrophobic material. Thus, a porous structure layer having a desired flow path structure can be more assuredly obtained.

The term "impregnation ratio" encompasses an impregnation ratio associated with the material **M'** in the region of the porous structure layer, which is formed of the material **M'** extended over the entire thickness direction. Moreover, the material M' obtained by immersing the material M in a hydrophobic material that is sufficiently heated to have a low viscosity (e.g., heated at 120°C), and leaving the resultant material to stand for a sufficient period (e.g., 3 minutes) while maintaining the temperature is determined as having an impregnation ratio of 100%. The impregnation ratio can be adjusted, for example, by adjusting an amount of the hydrophobic material used for impregnation (a thickness of a hydrophobic film, and the like).

A measurement method of the impregnation ratio of the hydrophobic material relative to the material M is not particularly limited, and may be appropriately selected according to the intended purpose. Examples of the measurement method include the following method.

[Measurement method of impregnation ratio]

Filter paper is cut into an appropriate size, and dried at 120°C for 3 minutes. Then, a dry mass M0 (g) of the filter paper is measured. Subsequently, the filter paper is impregnated with a hydrophobic material, and is left to stand at 120°C for 3 minutes. The impregnated filter paper is held between the same type of filter paper and glass slides, and is left to stand at 120°C for 1 minute with a load of 100 gf to remove the excess hydrophobic material. Thereafter, a mass M1 (g) of the filter paper is measured, and the maximum impregnation amount Pmax (g/m$^2$) per unit area is calculated according to the following equation.

$$\text{Equation: Pmax } (g/m^2) = (M1 - M0) \times 1,000$$

The viscosity of the hydrophobic material is not particularly limited, and may be appropriately selected according to the intended purpose. For example, for sufficiently avoiding a problem, such as blockage during the impregnation of the material **M,** the viscosity of the hydrophobic material at 140°C and at the shearing speed of 3,000 s$^{-1}$ is preferably 100 mPa·s or lower, more preferably 50 mPa·s or lower, and yet more preferably 30 mPa·s or lower. Although the measurement of the viscosity is not particularly limited, the viscosity can be measured, for example, by a rheometer (e.g., product name: AR-G2 rheometer, produced by TA Instruments Inc.).

The material M' is preferably colored so that a flow of a liquid can be easily visually recognized, but may be white or transparent, or may not be colored. The coloring of the material **M'** can be achieved, for example, by impregnating the material M with a colorant in addition to the hydrophobic material. Examples of the colorant include pigments, such as carbon black (a black pigment). The colorant is preferably hydrophobic. Moreover, as the colorant, a colorant that does not adversely affect a reagent used for an inspection or the like is preferably selected.

A shape of the porous structure layer 101 in a plan view is not particularly limited, and may be appropriately selected according to the intended purpose. Examples of the shape of the porous structure layer 101 include rectangular shapes, substantially circular shapes, substantially elliptical shapes, substantially rectangular shapes, and the like.

-Separation portion-

A separation portion is provided in the porous structure layer 101 of the sheet-like structure 11. The separation portion separates adjacent portions of the flow path of the porous structure layer 101 so that flow of a fluid in the flow path can be blocked. Moreover, the separation portion preferably separates the fluid inlet portion from the flow path adjacent to the fluid inlet portion so that the fluid entering the fluid inlet portion can be blocked from flowing into the flow path adjacent to the fluid inlet portion. More specifically, in the case where the flow path A in Figs. 1A and 1B is a fluid inlet portion, for example, the separation portion preferably separates the flow path A from the flow path C adjacent to the flow path A so that the fluid entering the flow path A can be blocked from flowing into the flow path **C.**

The phrase "separating adjacent portions of the flow path to block flow of the fluid in the flow path" encompasses that the porous structure constituting the flow path is separated at the separation portion, thereby creating a state in which flow of a fluid does not occur.

A position at which the separation portion is arranged is not particularly limited as long as the separation portion can separate adjacent portions of the flow path. The separation portion is preferably a region between the fluid inlet portion and the detection portion, and the region other than the fluid inlet portion and the detection portion.

Moreover, the number of the separation portions is not particularly limited, and can be appropriately set according to the intended purpose. Multiple reactions (multistage reaction) can be performed within one sheet-like structure by increasing the number of the separation portions.

The separation portion is not particularly limited as long as the separation portion can separate the adjacent portions of the flow path, and may be appropriately selected according to the intended purpose. The separation portion is preferably a cut portion. The cut portion is a region that cuts off the flow path in the direction intersecting the flow direction of a fluid in the flow path as illustrated in Figs. 1A and 1B. The "direction intersecting the flow direction of a fluid" is not particularly limited, and may be, for example, a direction perpendicular or substantially perpendicular to the flow direction of a fluid, as illustrated in Fig. 1A.

As an embodiment in which the separation portion is not the cut portion, the separation portion can be, for example, a region where the porous structure is stretched in the vertical direction or the horizontal direction (horizontal direction in Fig. 1A) to separate the porous structures in the porous structure layer. In this embodiment, a below-described support is preferably a material **(e.g.,** an elastic member) that can be stretched in the vertical direction or the horizontal direction.

In the case where the separation portion is the cut portion, the cut portion is preferably present only in the porous structure layer of the sheet-like structure, and is not present in a below-described support layer. In other words, the cut portion is preferably arranged so that, when the sheet-like structure is separated at the cut portion, the porous structure layer is separated, but the support layer is not separated. Since the support layer remains connected without being separated, the support layer functions as a hinge, so that the separated portions of the flow path are brought back to be in contact with each other again to allow flow of the fluid to continue.

The function of the separation portion will be specifically described with reference to Figs. 2A and 2B.

Fig. 2A is an explanatory view for explaining an example of the function of the separation portion. Specifically, Fig. 2A is a view illustrating a state in which the sheet-like structure 11 illustrated in Fig. 1A is folded by approximately 45° at the separation portion X. Fig. 2B is an explanatory view for explaining another example of the function of the separation portion. Specifically, Fig. 2B is a view illustrating a state in which the sheet-like structure 11 illustrated in Fig. 1A is folded by approximately 180° at the separation portion X. In both the states of Figs. 2A and 2B, the support layer 301 remains connected without being separated as described above.

In the both states of Figs. 2A and 2B, the porous structure of the porous structure layer 101 is separated, specifically, the adjacent portions of the flow path are separated, thereby blocking flow of a fluid in the flow path. More specifically, in the case where the flow path A is the fluid inlet portion, for example, the flow path A and the flow path C adjacent to the flow path A are separated by the separation portion so that a fluid entering the flow path A is blocked from flowing into the flow path C.

Since the sheet-like structure 11 has the above structure, flow of a fluid does not occur in the flow path, and the fluid can be sufficiently retained in the fluid inlet portion. Since the support layer 301 is not separated by the separation portion and functions as a hinge as described above, after retaining the fluid for a predetermined period, the separated portions of the flow path can be brought into contact with each other again, thereby allowing flow of the fluid to continue as illustrated in Fig. 1B.

In the case where the sheet-like structure 11 is used as an inspection device, for example, a test sample and a reagent that reacts with the test sample (e.g., an antigen and antibody, etc.) are introduced into the fluid inlet portion of the sheet-like structure in the state of Fig. 2A or 2B so that a sufficient reaction time between the test sample and the reagent can be ensured. Further, after completing the reaction (or after a predetermined time has elapsed), the separated portions of the flow path are brought into contact with each other again as illustrated in Fig. 1B, thereby allowing flow of the test sample and the reagent to continue.

Since the sheet-like structure 11 has the above configuration, moreover, flow of the fluid entering the fluid inlet portion can be regulated. Specifically, the fluid can be inhibited from being unevenly distributed due to the force of the fluid at the time of entering by temporarily separating the portions of the flow path by the separation portion.

An angle at which the sheet-like structure is folded at the separation portion X is not particularly limited as long as the angle is an angle at which the portions of the flow path can be separated to block flow of a fluid, and may be appropriately selected according to the intended purpose.

-Support layer-

The sheet-like structure 11 includes a support layer 301.

As described above, the support layer 301 functions as a hinge of the sheet-like structure 11, thereby bringing the separated portions of the flow path into contact with each other again to allow flow of the fluid to continue. Further, the support layer also has a function of reinforcing the physical strength of the sheet-like structure itself.

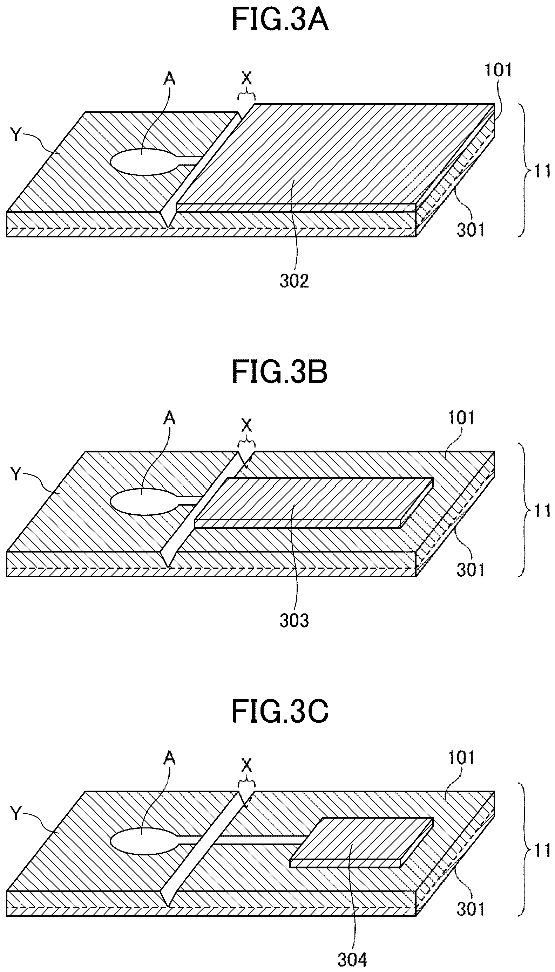

The distribution of the support layer will be specifically described with reference to Figs. 3A to 3C. Fig. 3A is an explanatory view for explaining an example of the distribution of the support layer. Fig. 3B is an explanatory view for explaining another example of the distribution of the support layer. Fig. 3C is an explanatory view for explaining yet another example of the distribution of the support layer.

The support layer of the sheet-like structure can be arranged in a region of the porous structure layer where the fluid inlet portion is not positioned. For example, as illustrated in Figs. 1A and 1B, the support layer may be provided only on the surface of the sheet-like structure from which a fluid is not allowed to enter the fluid inlet portion. As illustrated in Fig. 3A, the support layer may be provided on the both surfaces of the porous structure layer. As illustrated in Figs. 3B and 3C, the support layer may be provided to cover the exposed flow path, or part of the flow path. In the case where the support layer is provided on the both surfaces of the porous structure layer, particularly in the case where the support layer is provided on the surface of the porous structure layer from which a fluid is allowed to enter the fluid inlet portion, the support layer is preferably arranged not to overlap the separation portion.

Since the sheet-like structure has the above configuration, contamination of a fluid flowing through the flow path can be avoided. In the case where the fluid is a test sample and a reagent (e.g., an antigen and an antibody, and the like), for example, the sheet-like structure having the above configuration is suitable because inhibition of a reaction due to contamination or the like can be avoided.

The support layer is preferably impermeable to water. A material of the support layer that is impermeable to water is not particularly limited, and may be appropriately selected according to the intended purpose. Examples of the material include polypropylene and the like.

A size, structure, and shape of the support layer are not particularly limited as long as the support layer can cover the flow path of the porous structure layer, and may be appropriately selected according to the intended purpose.

As the support layer, a commercially available product can be used. Examples of the commercially available product include a product name of 660-PF (produced by NICHIBAN Co., Ltd.) and the like.

The average thickness of the sheet-like structure 11 of the first embodiment is not particularly limited, and may be appropriately selected according to the intended purpose. For example, the average thickness of the sheet-like structure 11 can be 100 $\mu$m or greater and 300 $\mu$m or less. The average thickness of the first sheet-like structure 11 can be measured, for example, by a thickness gauge under a product name of id-c112bs (produced by Mitutoyo Corporation).

A size of the sheet-like structure 11 of the first embodiment is not particularly limited, and may be appropriately selected according to the intended purpose.

<Second embodiment>

In the porous structure layer, the exposed shape of the flow path exposed at one surface of the porous structure layer and the exposed shape of the flow path exposed at the other surface of the porous structure layer may be different from each other. In other words, the porous structure layer may include two or more layers having mutually different flow path shapes.

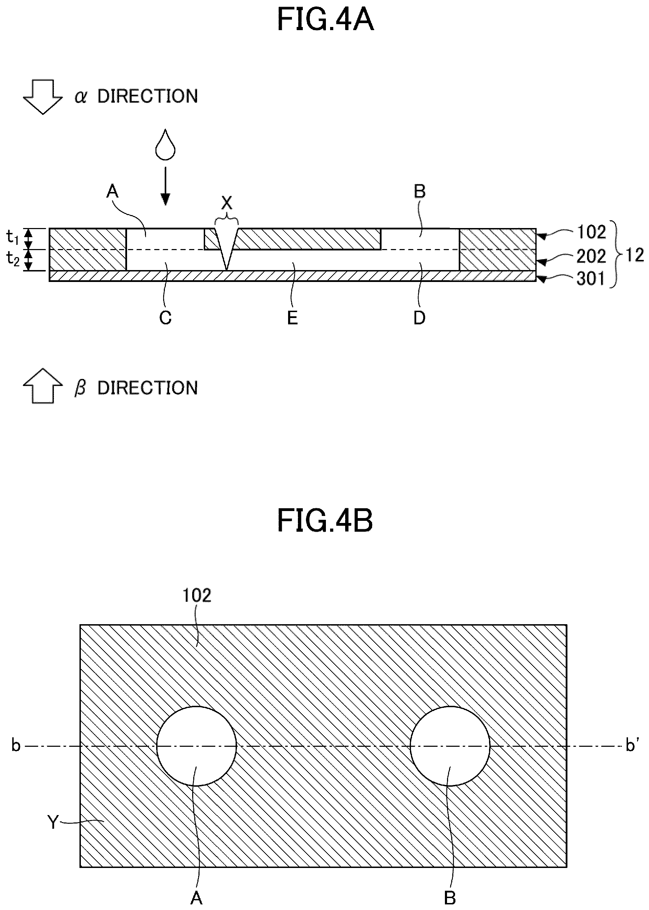

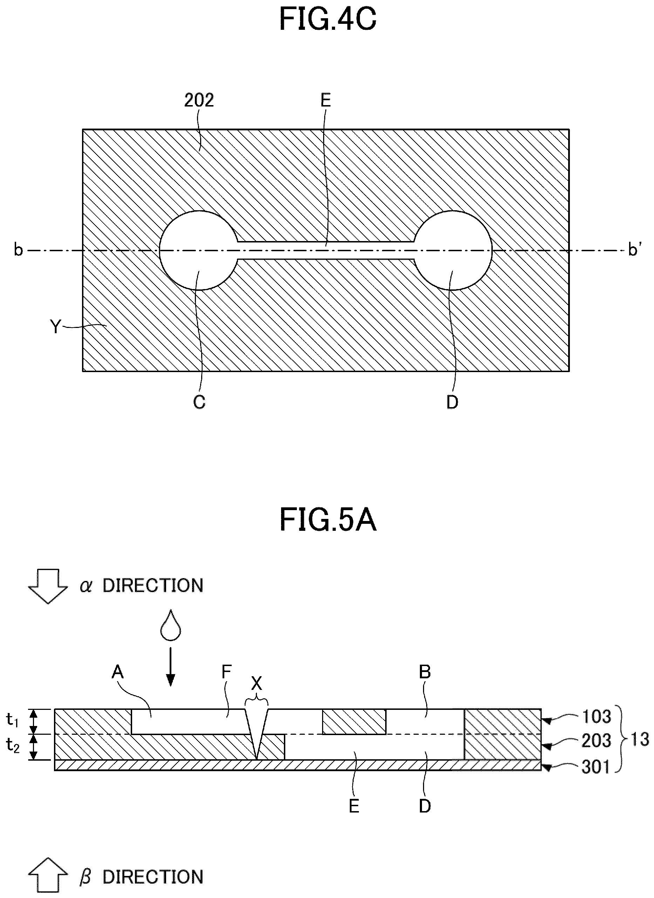

The sheet-like structure 12 of the second embodiment will be specifically described with reference to Figs. 4A to 4C. Fig. 4A is a schematic cross-sectional view of the sheet-like structure of the second embodiment. Fig. 4B is a schematic plan view of the porous structure layer of Fig. 4A as viewed in the α direction. Fig. 4C is a schematic plan view of the porous structure layer (i.e., the first porous structure layer 102 and the second porous structure layer 202, which are the structure of the sheet-like structure 12 from which the support layer 301 is removed) of Fig. 4A as viewed in the β direction. Moreover, Fig. 4A is a schematic cross-sectional view cut along the line b-b' illustrated in Figs. 4B and 4C. Further, the illustration of the separation portion X is omitted in Figs. 4B and 4C.

In the first porous structure layer 102 of the sheet-like structure 12 of the second embodiment, a flow path A, a flow path B, and a non-flow path Y that is a region other than the flow paths A and B are provided. The flow path A and the flow path B are separated in the first porous structure layer 102.

In the second porous structure layer 202 of the sheet-like structure 12 of the second embodiment, a flow path C, a flow path D, a flow path E, and a non-flow path Y that is a region other than the flow paths C, D, and E are provided. The flow path E is connected to both the flow path C and the flow path D, and a fluid can flow in the order of the flow path C, the flow path E, and the flow path D, or in the order of the flow path D, the flow path E, and the flow path C.

In the sheet-like structure 12 of the second embodiment, a support layer 301 is provided on the second porous structure layer 202 side of the sheet-like structure 12.

Note that nothing is interposed between the first porous structure layer 102 and the second porous structure layer 202, and the first porous structure layer 102 and the second porous structure layer 202 are adjacent to each other.

In the sheet-like structure 12 of the second embodiment, the flow path A of the first porous structure layer 102 and the flow path C of the second porous structure layer 202 are adjacent to each other, and the flow path B of the first porous structure layer 102 and the flow path D of the second porous structure layer 202 are adjacent to each other. Specifically, in the sheet-like structure 12 of the second embodiment, the flow paths are adjacent to one another and connected in the order of the flow path A, the flow path C, the flow path E, the flow path D, and the flow path B, or in the order of the flow path B, the flow path D, the flow path E, the flow path C, and the flow path A.

In the sheet-like structure 12 of the second embodiment, a separation portion X is formed to block between the flow path C and the flow path E.

Moreover, in Fig. 4A, an edge region when a hydrophobic material is allowed to penetrate from the first porous structure layer 102 side is illustrated with a dotted line as an imaginary line. Similarly, an edge region when a hydrophobic material is allowed to penetrate from the second porous structure layer 202 side is illustrated with a dotted line as an imaginary line. The imaginary lines are illustrated similarly in other drawings.

Further, the flow path A, the flow path B, the flow path C, the flow path D, the flow path E, and the non-flow path Y are formed of the material M or the material M' described in the above section of <First embodiment>.

In the sheet-like structure 12 of the second embodiment, the flow paths are adjacent to one another and connected in the order of the flow path A, the flow path C, the flow path E, the flow path D, and the flow path B, or in the order of the flow path B, the flow path D, the flow path E, the flow path C, and the flow path A, as illustrated in Fig. 4A. In other words, the sheet-like structure 12 of the second embodiment is configured, for example, such that when a fluid is allowed to drip into the flow path A, the fluid is passed through the flow path A, the flow path C, the flow path E, and the flow path D in this order due to a capillary action or the like, and is ultimately allowed to flow into the flow path B. In this case, the flow path A acts as the fluid inlet portion, and the flow path B acts as the detection portion.

In the sheet-like structure, a ratio (t2/t1) of the average thickness of the second porous structure layer (t2) to the average thickness (t1) of the first porous structure layer is preferably 0.56 or greater and 2.2 or less. By producing the sheet-like structure to have the average thickness ratio (t2/t1) of 0.56 or greater and 2.2 or less, a problem, such as blockage, can be sufficiently avoided when the material M is impregnated with the hydrophobic material, and a flow speed, speed stability, or both of a fluid, for example, from a flow path to another flow path, can be efficiently improved. From the same viewpoint, the average thickness ratio (t2/t1) is greater than **1.0.** Specifically, the average thickness (t2) of the second porous structure layer is more preferably greater than the average thickness (t1) of the first porous structure layer, yet more preferably 1.3 or greater, and particularly preferably 1.8 or greater. Moreover, the average thickness ratio (t2/t1) is not particularly limited, and may be set at 3.0 or less.

The average thickness of the sheet-like structure 12 of the second embodiment is not particularly limited, and may be appropriately selected according to the intended purpose. For example, the average thickness of the sheet-like structure 12 can be set to 100 μm or greater and 300 μm or less. The average thickness can be measured by a thickness gauge under a product name of id-c112bs (produced by Mitutoyo Corporation).

A size of the sheet-like structure 12 of the second embodiment is not particularly limited, and may be appropriately selected according to the intended purpose.

<Third embodiment>

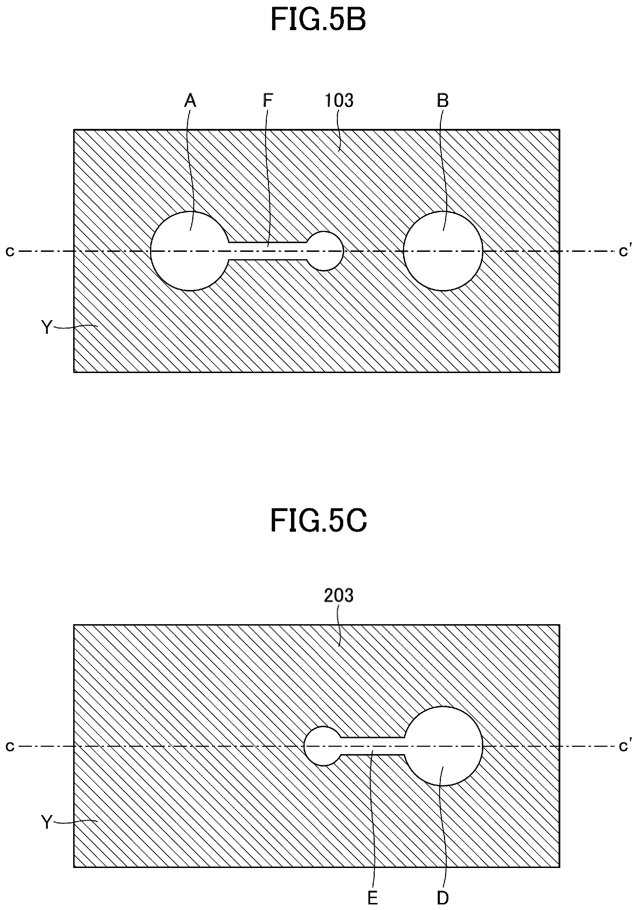

The sheet-like structure 13 of the third embodiment will be specifically described with reference to Figs. 5A to 5C. Fig. 5A is a schematic cross-sectional view of the sheet-like structure of the third embodiment. Fig. 5B is a schematic plan view of the porous structure layer of Fig. 5A as viewed in the α direction. Fig. 5C is a schematic plan view of the porous structure layer of Fig. 5A as viewed in the β direction. Note that the sheet-like structure 13 illustrated in Figs. 5A to 5C is substantially the same as the sheet-like structure 12 of the second embodiment, except that the flow path structure of the sheet-like structure is different from the flow path structure of the sheet-like structure of the second embodiment. Moreover, Fig. 5A is also a schematic cross-sectional view cut along the line c-c' illustrated in Figs. 5B and 5C. Further, the illustration of a separation portion X is omitted in Figs. 5B and 5C.

In the sheet-like structure 13 of the third embodiment, a flow path A, a flow path B, a flow path F connected to the flow path A, and a non-flow path Y that is a region other than the flow path F are provided in the first porous structure layer 103, as illustrated in Fig. 5B. The flow path A and the flow path B are separated in the first porous structure layer 103 of the sheet-like structure 13 of the third embodiment.

In the sheet-like structure 13 of the third embodiment, a flow path D, a flow path E, and a non-flow path Y that is a region other than the flow paths D and E are provided in the second porous structure layer 203 as illustrated in Fig. 5C, and the flow path E is connected to the flow path D.

In the sheet-like structure 13 of the third embodiment, a support layer 301 is disposed on the second porous structure layer 203 side of the sheet-like structure 13.

In the sheet-like structure 13 of the third embodiment, the flow path B of the first porous structure layer 103 and the flow path D of the second porous structure layer 203 are adjacent to each other, and the flow path F of the first porous structure layer 103 and the flow path E of the second porous structure layer 203 are connected to each other. Specifically, in the sheet-like structure 13 of the third embodiment, the flow paths are adjacent to one another and are connected in the order of the flow path A, the flow path F, the flow path E, the flow path D, and the flow path B, or in the order of the flow path B, the flow path D, the flow path E, the flow path F, and the flow path A.

In the sheet-like structure 13 of the third embodiment, the separation portion X is formed to block between the flow path F and the flow path E.

Further, the flow path A, the flow path B, the flow path D, the flow path E, the flow path F, and the non-flow path Y are formed of the material M or the material M' described in the above section of <First embodiment>.

As illustrated in Fig. 5A, the sheet-like structure 13 of the third embodiment is configured, for example, such that when a fluid is allowed to drip into the flow path A, the fluid is passed through the flow path A, the flow path F, the flow path E, and the flow path D in this order due to a capillary action or the like, and is ultimately allowed to flow into the flow path B. In this case, the flow path A acts as the fluid inlet portion, and the flow path B acts as the detection portion.

Other than above, descriptions of the same features as in

<First embodiment> will be omitted.

<Fourth embodiment>

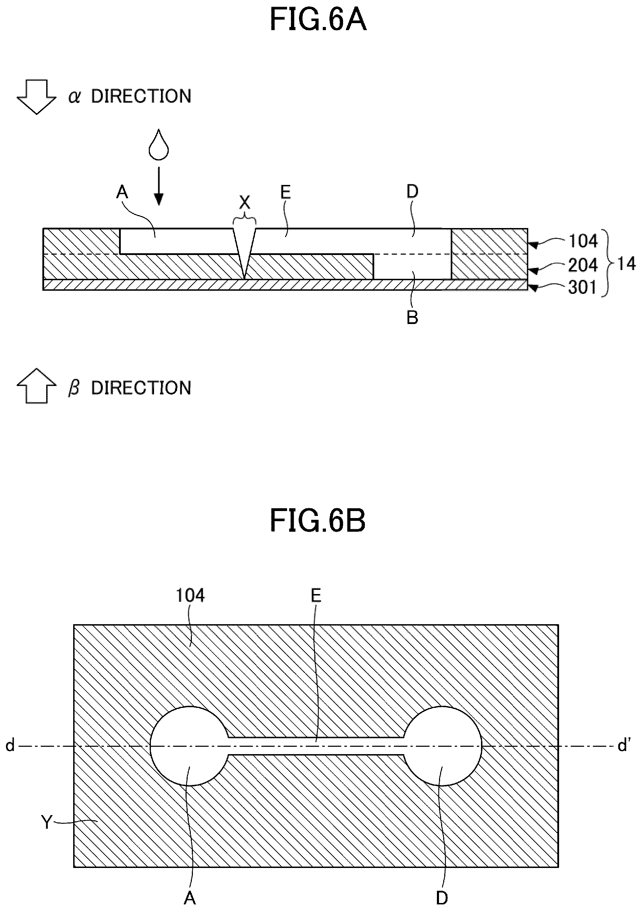

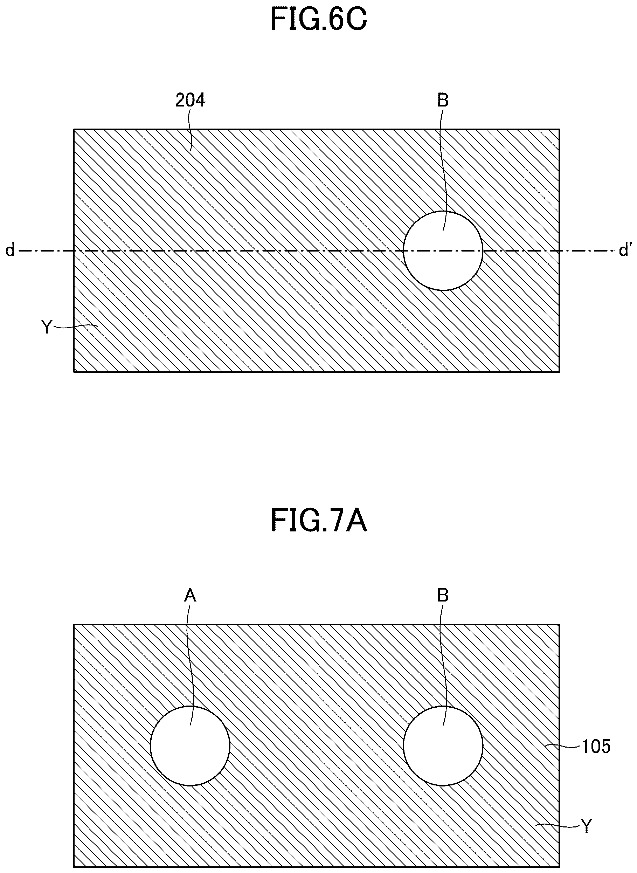

The sheet-like structure 14 of the fourth embodiment will be specifically described with reference to Figs. 6A to 6C. Fig. 6A is a schematic cross-sectional view of the sheet-like structure of the fourth embodiment. Fig. 6B is a schematic plan view of the porous structure layer of Fig. 6A as viewed in the α direction. Fig. 6C is a schematic plan view of the porous structure layer of Fig. 6A as viewed in the β direction. Note that the sheet-like structure 14 illustrated in Figs. 6A to 6C is substantially the same as the sheet-like structure 12 of the second embodiment, except that the flow path structure of the sheet-like structure is different from the flow path structure of the sheet-like structure of the second embodiment. Moreover, Fig. 6A is also a schematic cross-sectional view cut along the line d-d' illustrated in Figs. 6B and 6C.

In the sheet-like structure 14 of the fourth embodiment, a flow path B and a non-flow path Y that is a region other than the flow path B are provided in the first porous structure layer 104 as illustrated in Fig. 6B, and a flow path A is not provided.

In the sheet-like structure 14 of the fourth embodiment, a flow path A, a flow path D, a flow path E, and a non-flow path Y that is a region other than the flow paths A, E, and D are provided in the second porous structure layer 204, as illustrated in Fig. 6C. The flow path E is connected to both the flow path D and the flow path A.

In the sheet-like structure 14 of the fourth embodiment, a support layer 301 is disposed on the second porous structure layer 204 side of the sheet-like structure 14.

In the sheet-like structure 14 of the fourth embodiment, the flow path D of the first porous structure layer 104 and the flow path B of the second porous structure layer 204 are adjacent to each other. Specifically, the flow paths are adjacent to one another and are connected in the order of the flow path A, the flow path E, the flow path D, and the flow path B, or in the order of the flow path B, the flow path D, the flow path E, and the flow path A in the sheet-like structure 14 of the fourth embodiment.

In the sheet-like structure 14 of the fourth embodiment, a separation portion X is formed to block between the flow path A and the flow path E.

Further, the flow path A, the flow path B, the flow path D, the flow path E, and the non-flow path Y are formed of the material M or the material M' described in the above section of

<First embodiment>.

As illustrated in Fig. 6A, the sheet-like structure 14 of the fourth embodiment is configured, for example, such that when a fluid is allowed to drip into the flow path A, the fluid is passed through the flow path A, the flow path E, and the flow path D in this order due to a capillary action or the like, and is ultimately allowed to flow into the flow path B. In this case, the flow path A acts as the fluid inlet portion, and the flow path B acts as the detection portion.

Other than above, descriptions of the same features as in

<First embodiment> will be omitted.

<Fifth embodiment>

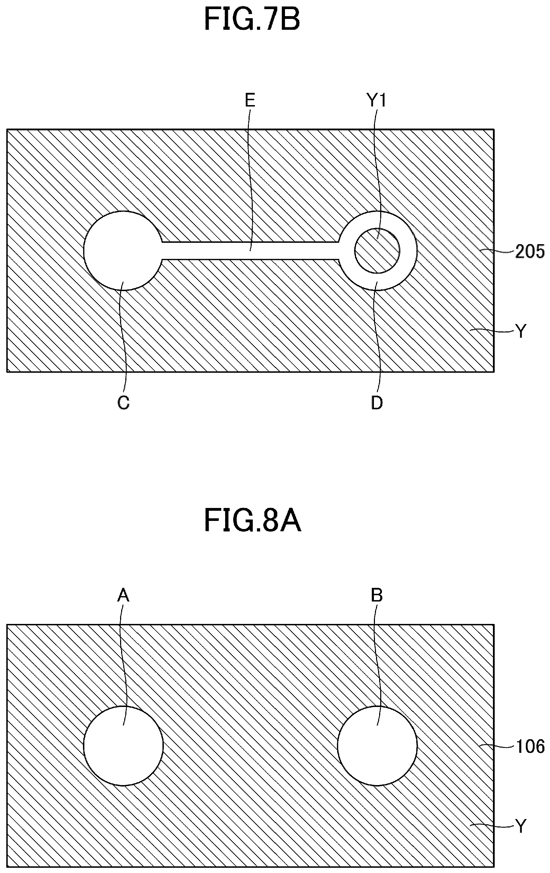

The sheet-like structure of the fifth embodiment will be specifically described with reference to Figs. 7A and 7B. Fig. 7A is a schematic plan view of the porous structure layer of the sheet-like structure of the fifth embodiment as viewed in the $\alpha$ direction. Fig. 7B is a schematic plan view of the porous structure layer of the sheet-like structure of the fifth embodiment as viewed in the $\beta$ direction.

As illustrated in Figs. 7A and 7B, the sheet-like structure of the fifth embodiment is the same as illustrated in Figs. 4B and 4C, except that the flow path D provided in the second porous structure layer 205 is an annular structure, and a non-flow path Y1 is formed inside the annular structure.

The flow path D, which is an annular structure, may have any outline shape, such as a circular shape, an elliptical shape, a rectangular shape, or the like. The flow path D preferably has an outline shape substantially matched with the flow path B in a plan view of the sheet-like structure. Moreover, the non-flow path Y formed inside the flow path D preferably has a shape obtained by reducing the outline shape of the flow path D in a plan view of the sheet-like structure.

<Sixth embodiment>

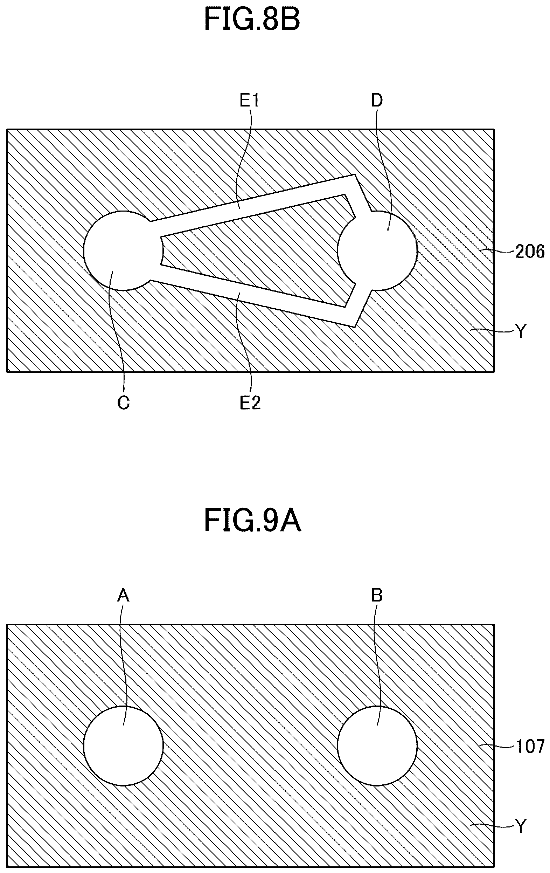

The sheet-like structure of the sixth embodiment will be specifically described with reference to Figs. 8A and 8B. Fig. 8A is a schematic plan view of the porous structure layer of the sheet-like structure of the sixth embodiment as viewed in the $\alpha$ direction. Fig. 8B is a schematic plan view of the porous structure layer of the sheet-like structure of the sixth embodiment as viewed in the $\beta$ direction.

As illustrated in Figs. 8A and 8B, the sheet-like structure of the sixth embodiment is the same as illustrated in Figs. 7A and 7B, except that the second porous structure layer 206 has a structure including multiple flow paths E (two paths E1 and E2 in Fig. 8B). In association with the above structure, multiple flow paths F can be provided to match the number of the flow paths E so that the multiple flow paths F of the first porous structure layer and the multiple flow paths E of the second porous structure layer are connected, respectively.

<Seventh embodiment>

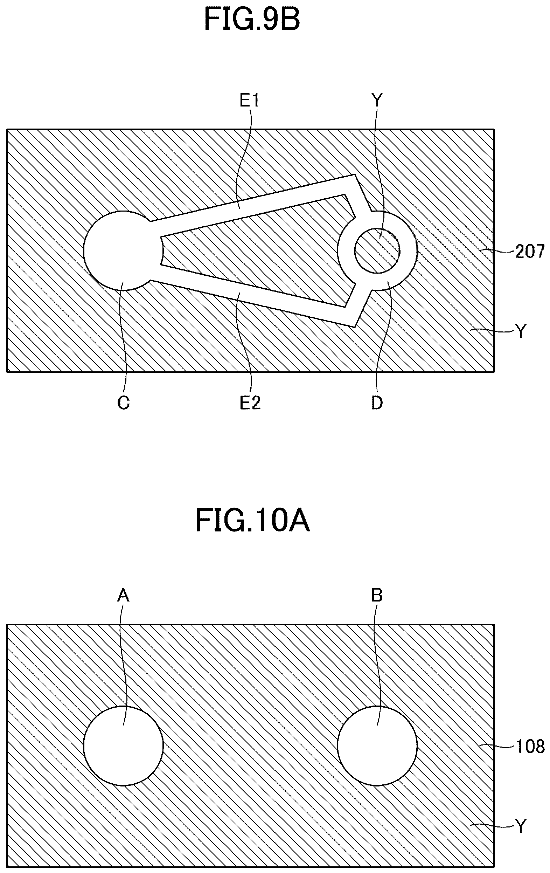

The sheet-like structure of the seventh embodiment will be specifically described with reference to Figs. 9A and 9B. Fig. 9A is a schematic plan view of the porous structure layer of the sheet-like structure of the seventh embodiment as viewed in the $\alpha$ direction. Fig. 9B is a schematic plan view of the porous structure layer of the sheet-like structure of the second embodiment as viewed in the $\beta$ direction.

As illustrated in Figs. 9A and 9B, the sheet-like structure of the seventh embodiment has a configuration such that the flow path shapes of Figs. 7A, 7B, 8A, and 8B are combined.

<Eighth embodiment>

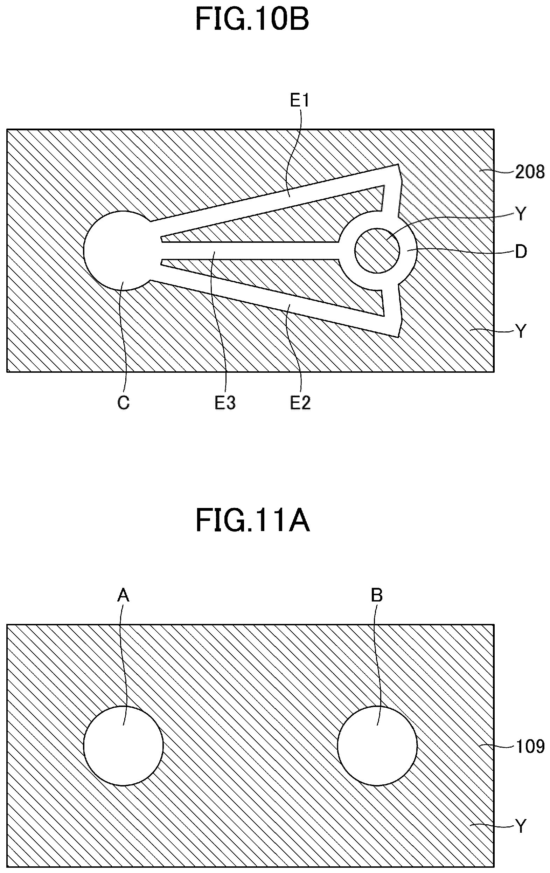

The sheet-like structure of the eighth embodiment will be specifically described with reference to Figs. 10A and 10B. Fig. 10A is a schematic plan view of the porous structure layer of the sheet-like structure of the eighth embodiment as viewed in the $\alpha$ direction. Fig. 10B is a schematic plan view of the porous structure layer of the sheet-like structure of the eighth embodiment as viewed in the $\beta$ direction.

As illustrated in Figs. 10A and 10B, the sheet-like structure of the eighth embodiment is substantially the same as illustrated in Fig. 9B, except that the second porous structure layer 208 has a structure including three flow paths E (E3 in addition to E1 and E2). In the sheet-like structure illustrated in Fig. 10B, the three flow paths E are connected to the flow path D in a manner such that the three flow paths E face one another in the flow path D.

<Ninth embodiment>

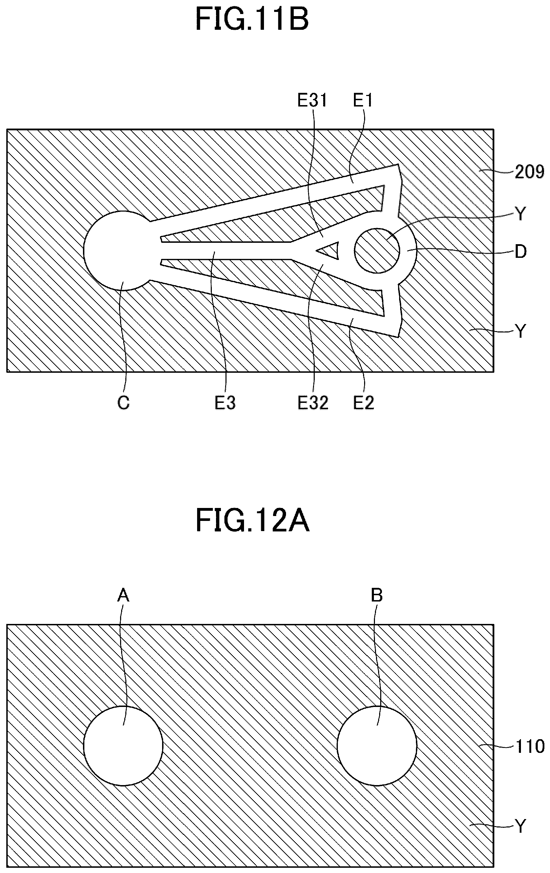

The sheet-like structure of the ninth embodiment will be specifically described with reference to Figs. 11A and 11B. Fig. 11A is a schematic plan view of the porous structure layer of the sheet-like structure of the ninth embodiment as viewed in the $\alpha$ direction. Fig. 11B is a schematic plan view of the porous structure layer of the sheet-like structure of the ninth embodiment as viewed in the $\beta$ direction.

As illustrated in Figs. 11A and 11B, the sheet-like structure of the ninth embodiment is substantially the same as illustrated in Fig. 10B, except that the flow path E3 is branched into two (E31 and E32), and the branched flow paths E31 and E32 are connected to the flow path D.

<Tenth embodiment>

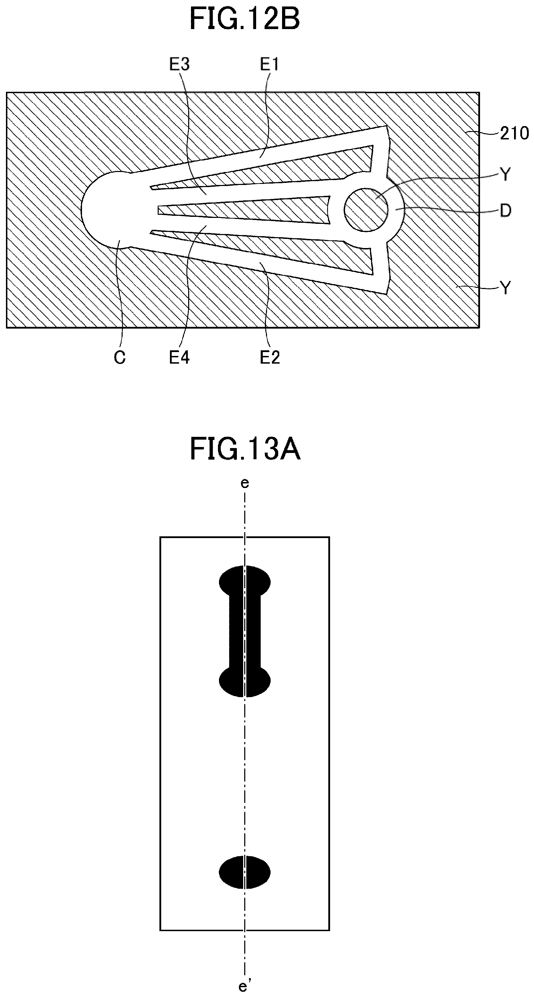

The sheet-like structure of the tenth embodiment will be specifically described with reference to Figs. 12A and 12B. Fig. 12A is a schematic plan view of the porous structure layer of the sheet-like structure of the tenth embodiment as viewed in the $\alpha$ direction. Fig. 12B is a schematic plan view of the porous structure layer of the sheet-like structure of the tenth embodiment as viewed in the $\beta$ direction.

As illustrated in Figs. 12A and 12B, the sheet-like structure of the tenth embodiment is substantially the same as illustrated in Fig. 11B, except that the sheet-like structure of the tenth embodiment has a structure including two flow paths E (E3 and E4) in addition to the flow path E1 and the flow path E2. In the sheet-like structure illustrated in Fig. 12B, the four flow paths E are connected to the flow path D in a manner such that the four flow paths E face one another in the flow path D.

The number of the flow paths E (and the flow paths F) in the sheet-like structure as illustrated in Figs. 8A to 12B is preferably 4 or less, more preferably 3 or less, and yet more preferably 2 in view of inhibition of increase in an amount of a fluid. Moreover, the number of connection points of the flow paths E with respect to the flow path D is preferably 4 or less, more preferably 3 or less, and yet more preferably **2.**

Among the multiple flow paths **E,** at least two flow paths E are preferably connected to the flow path D in a manner such that the flow paths E connected to the flow path D face each other. Moreover, among the multiple flow paths **E,** at least two flow paths E preferably have substantially the same shapes.

The above-described sheet-like structure can be produced, for example, by forming a predetermined portion (flow path B and the like) in a sheet-like material to produce a first porous structure layer, forming a predetermined portion (flow path D and the like) in another sheet-like material to produce a second porous structure layer, and laminating the above two sheet-like materials. Alternatively, the above-described sheet-like structure can also be produced by forming a predetermined portion in a part of one sheet-like material to produce a first porous structure layer, forming a predetermined portion in another part of the sheet-like material to produce a second porous structure layer, and folding the sheet-like material while adjusting the positions of the first porous structure layer and the second porous structure layer.

The sheet-like structure of the present invention is preferably produced by forming a first porous structure layer on one side of a sheet-like material, and forming a second porous structure layer on the other side of the sheet-like material. The above sheet-like structure in which the first porous structure layer and the second porous structure layer are respectively formed on corresponding sides of the sheet-like material has various advantages, such as (1) the labor and cost for laminating (or folding) can be avoided, (2) flow of a fluid between the first porous structure layer and the second porous structure layer by a capillary action can be ensured, (3) disposal is easily performed because a tool for retaining the laminating (folding) of the sheet-like materials or the like is not necessary, and the like.

As a specific production method of the sheet-like structure, for example, the sheet-like structure can be produced by the following method.

[One example of method of producing sheet-like structure]

First, a hydrophobic material, a colorant, and a resin are blended. The resultant mixture is melted and mixed, for example, at 100°C or higher and 140°C or lower, thereby preparing a wax ink. The wax ink is applied onto a substrate, such as a polyethylene terephthalate film, thereby producing an ink ribbon. Next, a predetermined flow path shape is printed on high quality paper by a thermal transfer printer (product name: L'esprit R412v-ex, produced by SATO HOLDINGS CORPORATION), thereby forming missing flow path patterns in the printed portions of the ink ribbon. After fixing pieces of the ink ribbon having the missing flow path patterns on corresponding front and back sides of filter paper, the resultant stack is passed through a thermal laminator (e.g., product name: GL535ML, produced by GBG) set at a predetermined temperature and line speed to transfer the wax ink to the filter paper and allow the wax ink to penetrate into the filter paper to form a three-dimensional flow path, thereby producing a sheet-like structure.

The filter paper is not particularly limited, and may be appropriately set according to the intended purpose. For example, filter paper having an average thickness of 310 $\mu$m, a basis weight of 94 g/m$^3$, and a capillary flow rate (CFR) of 13.9 sec/4 cm can be used.

The "predetermined temperature and line speed" are not particularly limited as long as the predetermined temperature and line speed are conditions under which the wax ink can penetrate and can be transferred to the filter paper. For transferring, the conditions can be set at 85°C and at the line speed of 10 mm/sec. For permeation, the conditions can be set at 85°C and at the line speed of 5 mm/sec.

(Method of using sheet-like structure)

The method of using the sheet-like structure of the present invention includes: allowing a fluid to enter a first flow path position at one side with respect to the separation portion; separating the first flow path from a second flow path at the separation portion to block flow of the fluid so that the fluid that has entered from the first flow path does not flow into the second flow path positioned at the other side with respect to the separation portion; and after a predetermined time has elapsed, bringing the first flow path and the second flow path into contact with each other at the separation portion to allow the fluid to flow between the first flow path and the second flow path.

In the method of using the sheet-like structure, the first flow path is preferably the fluid inlet portion, and the detection portion is preferably arranged at a part of the second flow path.

The method of using the sheet-like structure will be specifically described with reference to Figs. 1B, 2A, and 2B, hereinafter.

The "first flow path" is preferably, in other words, a portion of the flow path at the side where the fluid inlet portion from which a fluid is allowed to enter is present when the flow path is separated at the separation portion. Specifically, the first flow path is preferably a portion of the flow path including the flow path A, which is present on the left side with respect to the separation portion X in Fig. 1B.

The "second flow path" is preferably, in other words, the side of the flow path that is not the first flow path, specifically, a portion of the flow path at the side where the detection portion is present, when the flow path is separated at the separation portion. Specifically, the second flow path is preferably a portion of the flow path including the flow path B and the flow path C, which are present on the right side with respect to the separation portion X in Fig. 1B.

The phrase "separating the first flow path and the second flow path at the separation portion to block flow of the fluid" encompasses, for example, that portions of the flow path (porous structure) are separated at the separation portion as illustrated in Figs. 2A and 2B.

The "predetermined time" can be appropriately set according to viscosity of a fluid to be passed through, a reaction time of substances (e.g., an antigen and an antibody) included in the fluid, and the like.

The phrase "bringing the first flow path and the second flow path into contact with each other at the separation portion to allow the fluid to flow between the first flow path and the second flow path" encompasses, for example, that the state of the sheet-like structure illustrated in Fig. 2A or 2B is returned back to the state of Fig. 1B.

A blocking agent may be added to the fluid inlet portion and the detection portion in advance for the purpose of inhibition of flow and absorption of a fluid (a test sample such as an antigen, a reagent such as an antibody, etc.). Examples of the blocking agent include an albumin aqueous solution, and the like. The blocking agent is preferably appropriately selected according to a type of a fluid, or physical properties of a fluid, such as viscosity.

The sheet-like structure of the present invention can be suitably used as an inspection device. Examples of the inspection device include pregnancy test kits, inspection devices using a measuring method called an immunochromatography method in which the principle of sandwich ELISA and the principle of chromatography are combined, and the like.

Examples

Next, the present invention will be more specifically described through Examples and Comparative Examples, but the present invention is not limited to Examples below.

<Production of ink ribbon>

The following materials were blended, and the resultant mixture was melted and mixed at 100°C to prepare a wax ink. Paraffin wax as a hydrophobic material (product name: Paraffin Wax-135, produced by NIPPON SEIRO CO., LTD.): 72.0 parts by mass Synthetic wax as a hydrophobic material (product name: DIACARNA™ 30, produced by Mitsubishi Chemical Corporation): 18.0 parts by mass

Carbon black as a colorant (product name: MA-100, produced by Mitsubishi Chemical Corporation): 1.8 parts by mass Resin (product name: Ultrathene® 722, produced by Tosoh Corporation): 11.25 parts by mass

The viscosity of the obtained wax ink under the conditions of 140°C and the shearing speed of 3,000 $s^{-1}$ was 23 mPa·s. Note that the viscosity was measured by Rheometer AR-G2 (produced by TA Instruments Inc.).

The obtained wax ink was applied onto a polyethylene terephthalate film having the average thickness of 6 μm (product name: Lumirror® #6C F531, produced by TORAY INDUSTRIES, INC.) so that the average thickness of the applied ink was to be 5 μm to 12 μm, thereby producing an ink ribbon.

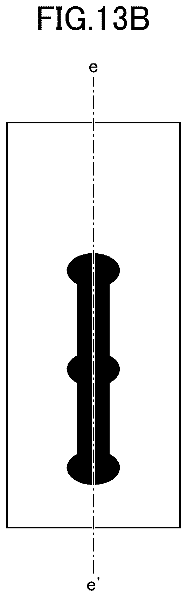

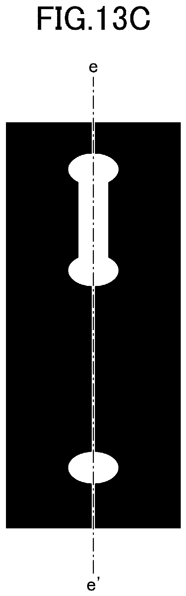

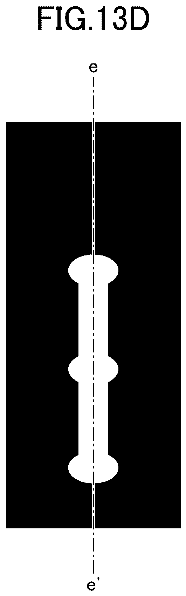

The flow path shapes illustrated in Figs. 13A and 13B were printed on high quality paper by a thermal transfer printer (product name: L'esprit R412v-ex, produced by SATO HOLDINGS CORPORATION), thereby forming missing flow path patterns in the printed portions of the ink ribbon (see Figs. 13C and 13D).

<Confirmation of flow path formation>

First, whether a flow path was appropriately formed was checked.

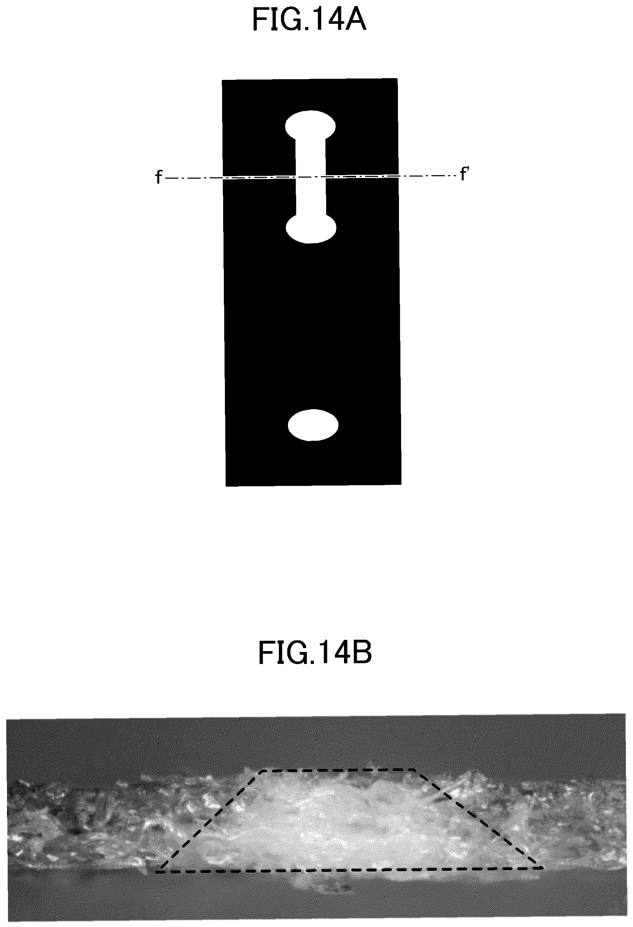

The flow path pattern as illustrated in Fig. 14A was printed on one side of filter paper (product name: Whatman #41) with the wax ink by a printer (product name: Xerox ColorQube8570, Xerox Corporation). Thereafter, the printed filter paper was heated in an oven under the conditions of 120°C for 2 minutes to allow the wax ink to penetrate into the filter paper, thereby forming a single-side printed flow path. After impregnating the single-side printed flow path with a fluorescent ink (fluorescent marker pen, produced by ASKUL Corporation) aqueous solution, the filter paper was cut at the position corresponding to the line f-f' of Fig. 14A, and a cross-sectional photograph of the flow path was captured by a microscope (produced by KEYENCE CORPORATION). The result is presented in Fig. 14B.

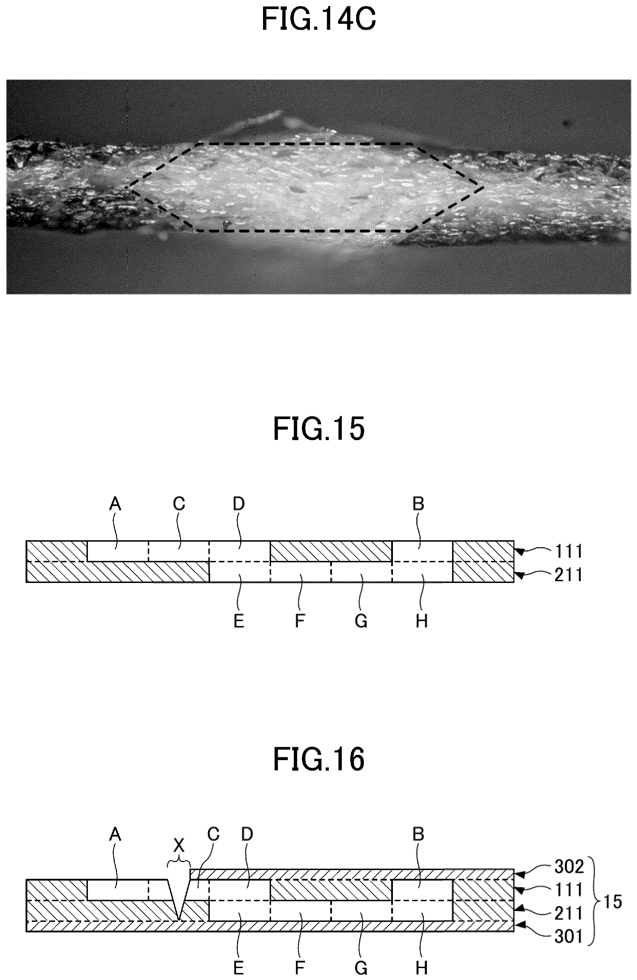

Similarly, a double-side printed flow path was formed within filter paper by performing impregnation under the same conditions, except that the flow path pattern illustrated in Fig. 14A was printed on both sides of filter paper (product name: Whatman #41). After impregnating the double-side printed flow path with a fluorescent ink (fluorescent marker pen, produced by ASKUL Corporation) aqueous solution, the filter paper was cut at the position corresponding to the line f-f' of Fig. 14A, and a cross-sectional photograph of the flow path was captured by a microscope (produced by KEYENCE CORPORATION). The result is presented in Fig. 14C.

The fluorescent ink was confirmed only in the portions each surrounded with the dotted line in Figs. 14B and 14C, and leakage of the fluorescent ink could not be observed in other regions. Therefore, it was confirmed that the flow path was appropriately formed. Since the single-side printing was performed, namely, that the wax ink penetrated from one side of the filter paper in Fig. 14B, the formed flow path had the shape that expanded from the plane from which the wax ink penetrated toward the plane of the opposite side, namely, a tapered shape. Since double-side printing was performed, namely, that the wax ink penetrated from both sides of the filter paper in Fig. 14C, the formed flow path became symmetric, and had a diamond shape.

<Quantitative analysis of ethyl paraoxon>

Ethyl paraoxon (or paraoxon) is a type of phosphorus-based agricultural chemicals. The paraoxon is a neurotoxin. If the paraoxon deposited on crops or the like enters the body, the paraoxon inhibits the function of acetylcholinesterase (AChE) involved in the neurotransmission, and causes convulsions, miosis, and the like. In Examples, a concentration of ethyl paraoxon in a test sample was quantitatively analyzed using the sheet-like structure.

-Production of porous structure layer-

Pieces of the ink ribbon on which missing flow path pattern illustrated in Figs. 13C and 13D were formed, respectively, were fixed on corresponding front and back sides of filter paper (average thickness: 310 μm, basis weight: 94 g/m³, capillary flow rate (CFR): 13.9 sec/4 cm). Thereafter, the resultant stack was passed through a thermal laminator (product name: GL535ML, produced by GBG) set at a predetermined temperature so that the wax ink was transferred to and permeated into the filter paper to form a three-dimensional flow path within the filter paper, thereby producing a sheet-like structure. The "predetermined temperature" was 85°C with the line speed of 10 mm/sec for transferring, and was 85°C with the line speed of 5 mm/sec for permeation.

The obtained porous structure layer had the structure illustrated in Fig. 15. Fig. 15 is a schematic cross-sectional view cut at the position corresponding to the line e-e' illustrated in Figs. 13A to 13D. For convenience, flow path names of flow paths A to H were assigned to the obtained porous structure layer as illustrated in Fig. 15. Moreover, the layer including the flow path A serving as the fluid inlet portion and the flow path B serving as the detection portion was represented as the first porous structure layer 111, and the layer that was not the first porous structure layer 111 was represented as the second porous structure layer 211.

-Blocking and fixing-

In order to inhibit absorption AChE (produced by Sigma-Aldrich) into the filter paper, 2 μL of a 0.8% albumin aqueous solution (produced by FUJIFILM Wako Pure Chemical Corporation) serving as a blocking agent was allowed to drip into the fluid inlet portion (flow path A), followed by drying at 30°C for 15 minutes.

Subsequently, 2 μL of a 0.25 (w/v%) polydiallyldimethylammonium chloride (PDDA) aqueous solution (produced by Sigma-Aldrich) serving as a fixing agent for indoxyl acetate (IDA) was allowed to drip into the detection portion (flow path B), followed by drying at 30°C for 15 minutes. Note that IDA is a substrate that reacts with AChE to generate a blue color.

Subsequently, after dissolving IDA (produced by Sigma-Aldrich) in methanol (produced by FUJIFILM Wako Pure Chemical Corporation), the resultant solution was diluted with pure, thereby preparing a solution in which a weight ratio of methanol to water was 70/30, and the concentration of IDA was 40 mM. The prepared solution in an amount of 3 μL was allowed to drip into the detection portion (flow path B), followed by drying at 30°C for 15 minutes.

-Production of support layer-

Pieces of an adhesive tape (product name: 660PF, produced by NICHIBAN Co., Ltd.) serving as support layers were stacked on the both sides of the support layer, respectively, so that the flow path A, which was the fluid inlet portion of the obtained porous structure, was not covered, and the resultant stacked layers were laminated by a hand roller at room temperature. For convenience, the support layer at the side of the first porous structure layer 111 was denoted by 302, and the support layer at the side of the second porous structure layer 211 was denoted by 301 as illustrated in Fig. 16.

Subsequently, a cut is formed between the fluid inlet portion (flow path A) and the flow path C adjacent to the fluid inlet portion by a cutter to form a separation portion X, thereby obtaining a sheet-like structure 15. During the cutting with the cutter, cutting was performed so as not to form a cut in the support layer 301. The obtained sheet-like structure 15 had the structure illustrated in Fig. 16.

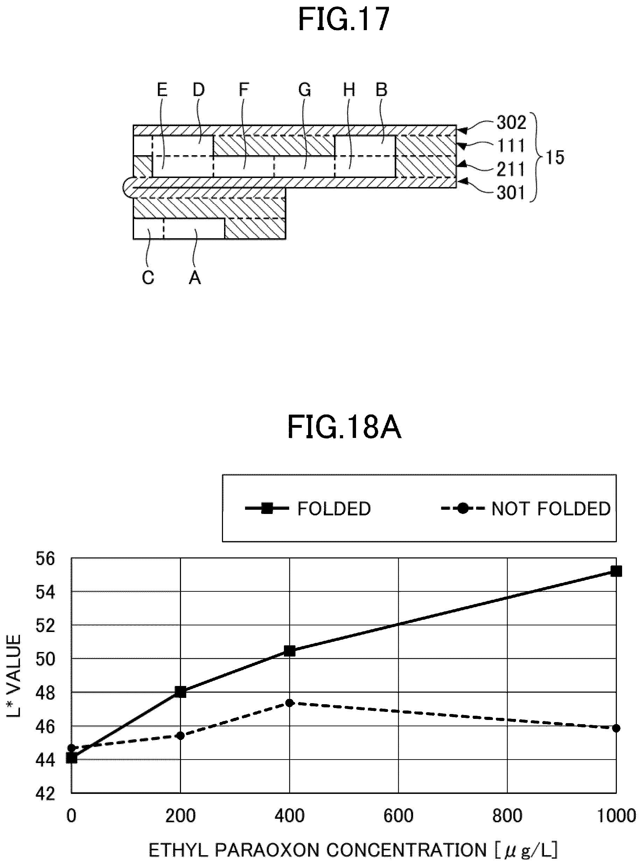

The obtained sheet-like structure 15 was folded at the separation portion X by 180° and was secured with a clip as illustrated in Fig. 17.

-Reaction-

A solution was prepared using Tris-HCl (pH 8.0) (produced by NIPPON GENE CO., LTD.) so that a concentration of AChE was to be 100 U/mL. The prepared solution in an amount of 3 μL was allowed to drip into the fluid inlet portion (flow path A), followed by drying at 30°C for 15 minutes.

Tris-HCl (pH 8.0), to which EtOH (produced by FUJIFILM Wako Pure Chemical Corporation) was added at a concentration of 6 vol%, was prepared, and the resultant Tris-HCL solution was used to prepare solutions having a concentration of ethyl paraoxon (produced by Sigma-Aldrich) of 0 μg/L, 200 μg/L, 400 μg/L, and 800 μg/L, respectively. Each of the prepared solutions was added to the fluid inlet portion (flow path A), and a reaction was allowed to occur at room temperature.

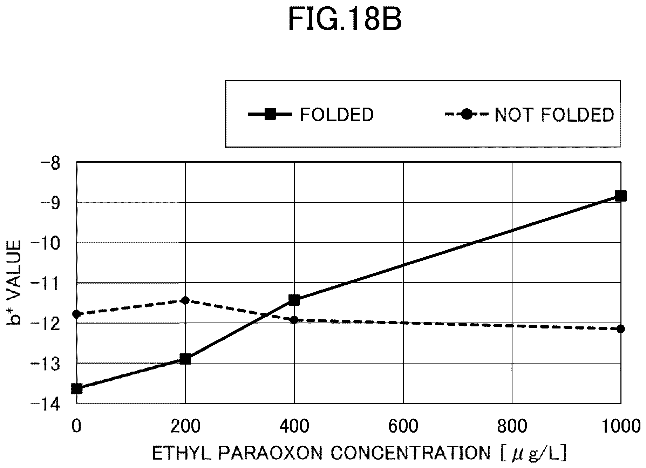

After leaving the resultant sheet-like structure 15 to stand for 5 minutes, the clip was removed, and the portions of the flow path of the sheet-like structure 15 were brought into contact with each other again (returning back to the state of Fig. 16), thereby allowing flow of the fluid to continue. After leaving the resultant sheet-like structure 15 to stand for 10 minutes, the developed color in the detection portion B was measured by a densitometer FD-05 produced by Konica Minolta, Inc., and the result was represented in the L*a*b* color system. The results are presented in Table 1, and Figs. 18A and 18B.

[Table 1]

| Sample concentration [$\mu$ g/L] | Folding | Sample 1 | | | Sample 2 | | | Sample 3 | | | Average values | | |
|---|---|---|---|---|---|---|---|---|---|---|---|---|---|
| | | L* | a* | b* | L* | a* | b* | L* | a* | b* | L* | a* | b* |
| 0 | Folded | 43.5 | -8.99 | -13.96 | 44.99 | -9.37 | -13.94 | 43.83 | -8.85 | -12.99 | 44.1 | -9.1 | -13.6 |
| 200 | | 47.98 | -9.59 | -12.48 | 50.93 | -9.97 | -12.21 | 45.17 | -9.36 | -13.99 | 48.0 | -9.6 | -12.9 |
| 400 | | 47.16 | -9.7 | -12.65 | 55.3 | -9.54 | -9.64 | 48.9 | -9.06 | -11.99 | 50.5 | -9.4 | -11.4 |
| 1000 | | 54.09 | -9.19 | -8.28 | 54.15 | -9.15 | -8.64 | 57.38 | -10.01 | -9.59 | 55.2 | -9.5 | -8.8 |
| 0 | Not folded | 44.81 | -9.28 | -11.6 | 45.74 | -8.95 | -11.49 | 43.48 | -9.12 | -12.25 | 44.7 | -9.1 | -11.8 |
| 200 | | 46.3 | -8.52 | -11.27 | 45.65 | -8.45 | -11.44 | 44.32 | -8.57 | -11.61 | 45.4 | -8.5 | -11.4 |
| 400 | | 48.1 | -9.21 | -11.23 | 47.82 | -9.14 | -12.08 | 46.2 | -8.97 | -12.46 | 47.4 | -9.1 | -11.9 |
| 1000 | | 45.63 | -8.85 | -11.75 | 45.15 | -8.92 | -12.35 | 46.82 | -8.73 | -12.34 | 45.9 | -8.8 | -12.1 |

The increase in the value of L* and the increase in the value of b* could be confirmed with the samples in the sheet-like structures folded at the separation portion compared to the sheet-like structures not folded at the separation portion. Specifically, the adjacent portions of the flow path could be completely blocked by folding the sheet-like structure at the separation portion so that a sufficient reaction time of the ethyl paraoxon and the AChE could be ensured, thereby improving the accuracy of the quantitative analysis.

The present application claims priority based on Japanese Patent Application No. 2022-188860, filed on November 28, 2022, the entire content of which are incorporated herein by reference.

REFERENCE SIGNS LIST

| | |
|---|---|
| 11 | first sheet-like structure of first embodiment |
| 12 | first sheet-like structure of second embodiment |
| 13 | first sheet-like structure of third embodiment |
| 14 | first sheet-like structure of fourth embodiment |
| 15 | first sheet-like structure of fifth embodiment |
| 16 | first sheet-like structure of sixth embodiment |
| 17 | first sheet-like structure of seventh embodiment |
| 18 | first sheet-like structure of eighth embodiment |
| 19 | first sheet-like structure of ninth embodiment |
| 101 | porous structure layer |
| 102 | first porous structure layer |
| 103 | first porous structure layer |
| 104 | first porous structure layer |
| 105 | first porous structure layer |
| 106 | first porous structure layer |
| 107 | first porous structure layer |
| 108 | first porous structure layer |
| 109 | first porous structure layer |
| 110 | first porous structure layer |
| 111 | first porous structure layer |
| 202 | second porous structure layer |
| 203 | second porous structure layer |
| 204 | second porous structure layer |
| 205 | second porous structure layer |
| 206 | second porous structure layer |
| 207 | second porous structure layer |
| 208 | second porous structure layer |
| 209 | second porous structure layer |
| 210 | second porous structure layer |
| 211 | second porous structure layer |
| 301 | support layer |
| 302 | support layer |
| 303 | support layer |
| 304 | support layer |
| A | flow path |
| B | flow path |
| C | flow path |
| D | flow path |
| E | flow path |
| E1 | flow path |
| E2 | flow path |
| E3 | flow path |
| E31 | flow path |
| E32 | flow path |
| E4 | flow path |
| F | flow path |

G      flow path
H      flow path
X1     flow path
X2     flow path
Y      non-flow path
Y1     non-flow path
M     material
M'     material
X      separation portion

**Claims**

1. A sheet-like structure comprising:

   a porous structure layer in which at least a part of a flow path having a porous structure capable of passing a fluid through is exposed at a surface of the porous structure layer;
   and
   a support layer,

   wherein the flow path has a separation portion capable of separating adjacent portions of the flow path to block flow of the fluid in the flow path.

2. The sheet-like structure according to claim 1,
   wherein the separation portion is a cut portion that cuts off the flow path in a direction intersecting with a flow direction of the fluid in the flow path.

3. The sheet-like structure according to claim 1 or 2,
   wherein at least a part of the part of the flow path exposed to the surface is a fluid inlet portion.

4. The sheet-like structure according to claim 3,
   wherein the separation portion is capable of separating the fluid inlet portion from a portion of the flow path adjacent to the fluid inlet portion so that a fluid that has entered from the fluid inlet portion is blocked from flowing into the portion of the flow path adjacent to the fluid inlet portion.

5. The sheet-like structure according to claim 3 or 4,
   wherein the support layer is arranged on a region of the porous structure layer in which the fluid inlet portion is not located.

6. The sheet-like structure according to any one of claims 1 to 5,
   wherein the support layer is impermeable to water.

7. The sheet-like structure according to any one of claims 1 to 6,
   wherein an exposed shape of the flow path that is exposed at one surface of the porous structure layer is different from an exposed shape of the flow path that is exposed at the other surface of the porous structure layer.

8. The sheet-like structure according to any one of claims 3 to 5,
   wherein at least part of the part of the flow path exposed at the surface is a detection portion, and the detection portion is arranged in a position of the flow path different from a position of the fluid inlet portion.

9. A method of using the sheet-like structure of any one of claims 1 to 8, the method comprising:

   allowing a fluid to enter a first flow path position at one side with respect to the separation portion;
   separating the first flow path from a second flow path at the separation portion to block flow of the fluid so that the fluid that has entered from the first flow path does not flow into the second flow path positioned at the other side with respect to the separation portion; and
   after a predetermined time has elapsed, bringing the first flow path and the second flow path into contact with each other at the separation portion to allow the fluid to flow between the first flow path and the second flow path.

**10.** The method of using the sheet-like structure according to claim 9,
wherein the first flow path is a fluid inlet portion, and a detection portion is arranged at a part of the second flow path.

# FIG.1A

# FIG.1B

# FIG.2A

# FIG.2B

# FIG.3A

# FIG.3B

# FIG.3C

# FIG.4A

α DIRECTION

A    X    B

t₁
t₂

102
202   12
301

C    E    D

β DIRECTION

# FIG.4B

102

b —·—·—·—·—·—·—·—·—·—·—·—·—·—·—·—·— b'

Y

A    B

# FIG.4C

202　　　　　　E

b —·—·—·———·—·—·— b'

Y

C　　　　　　　　D

# FIG.5A

⬇ α DIRECTION

💧

A　　　　F　　X　　　　B

$t_1$

$t_2$

E　　　D

103
203　}13
301

⬆ β DIRECTION

# FIG.5B

# FIG.5C

# FIG.6A

α DIRECTION

A       X     E        D

104
204 } 14
301

B

β DIRECTION

# FIG.6B

104       E

d — — — — — — — — — — — — — d'

Y

A          D

# FIG.6C

204          B

d —·—·——————————————————————— d'

Y

# FIG.7A

A          B

105

Y

# FIG.7B

# FIG.8A

EP 4 628 902 A1

# FIG.8B

# FIG.9A

28

# FIG.9B

E1    Y

C    E2    D

207

Y

# FIG.10A

A    B

108

Y

# FIG.10B

E1

208

Y

D

Y

C          E3          E2

# FIG.11A

A                    B

109

Y

# FIG.11B

# FIG.12A

# FIG.12B

# FIG.13A

# FIG.13B

# FIG.13C

# FIG.13D

## FIG.14A

## FIG.14B

# FIG.14C

# FIG.15

# FIG.16

# FIG.17

# FIG.18A

# FIG.18B

## INTERNATIONAL SEARCH REPORT

| International application No. |
|---|
| **PCT/JP2023/039971** |

### A. CLASSIFICATION OF SUBJECT MATTER

*G01N 35/08*(2006.01)i; *B01J 19/00*(2006.01)i; *B32B 5/18*(2006.01)i; *G01N 37/00*(2006.01)i
FI:   G01N35/08 A; G01N37/00 101; B01J19/00 321; B32B5/18

According to International Patent Classification (IPC) or to both national classification and IPC

### B. FIELDS SEARCHED

Minimum documentation searched (classification system followed by classification symbols)

G01N35/08; B01J19/00; B32B5/18; G01N37/00; F28F3/12

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2009-541037 A (MICROLYTIC APS) 26 November 2009 (2009-11-26) | 1-7 |
| | paragraphs [0064], [0164], [0169]-[0172], fig. 1, 2a-2b, 4b-4d | |
| Y | paragraphs [0064], [0164], [0169]-[0172], fig. 1, 2a-2b, 4b-4d | 8 |
| Y | JP 2003-139660 A (KAWAMURA INST OF CHEM RES) 14 May 2003 (2003-05-14) | 8 |
| | paragraph [0024] | |
| Y | JP 2002-219697 A (KAWAMURA INST OF CHEM RES) 06 August 2002 (2002-08-06) | 8 |
| | paragraph [0028] | |
| A | JP 2005-083510 A (TOSHIBA CORP) 31 March 2005 (2005-03-31) | 1-10 |
| A | JP 2016-003922 A (ENPLAS CORP) 12 January 2016 (2016-01-12) | 1-10 |
| A | JP 2011-145236 A (SEIKO EPSON CORP) 28 July 2011 (2011-07-28) | 1-10 |

☐ Further documents are listed in the continuation of Box C.       ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **10 January 2024** | **23 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2023/039971**

| Patent document cited in search report | | | Publication date (day/month/year) | Patent family member(s) | | | Publication date (day/month/year) |
|---|---|---|---|---|---|---|---|
| JP | 2009-541037 | A | 26 November 2009 | US | 2009/0264632 | A1 | |
| | | | | paragraphs [0072], [0207]-[0210], [0220]-[0223], fig. 1, 2a-2b, 4b-4d | | | |
| | | | | WO | 2008/000276 | A2 | |
| | | | | EP | 2032257 | A2 | |
| | | | | CA | 2653719 | A | |
| | | | | CN | 101479041 | A | |
| | | | | AU | 2007264161 | A | |
| JP | 2003-139660 | A | 14 May 2003 | (Family: none) | | | |
| JP | 2002-219697 | A | 06 August 2002 | US | 2003/0190265 | A1 | |
| | | | | paragraph [0052] | | | |
| | | | | WO | 2002/024320 | A1 | |
| | | | | EP | 1327474 | A1 | |
| | | | | AU | 3602001 | A | |
| | | | | CA | 2422550 | A1 | |
| | | | | CN | 1460036 | A | |
| | | | | KR | 10-2003-0038739 | A | |
| JP | 2005-083510 | A | 31 March 2005 | (Family: none) | | | |
| JP | 2016-003922 | A | 12 January 2016 | US | 2015/0360223 | A1 | |
| | | | | EP | 2957343 | A1 | |
| JP | 2011-145236 | A | 28 July 2011 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021175970 A
- JP 2022188860 A

**Non-patent literature cited in the description**

- **QUOC TRUNG HUA et al.** *analytical sciences*, April 2019, vol. 35, 393-399